Europäisches Patentamt

European Patent Office

Office européen des brevets

(19)

(11) Publication number: **0 039 217**

**A1**

(12)                        **EUROPEAN PATENT APPLICATION**

(21) Application number: **81301815.7**

(22) Date of filing: **24.04.81**

(51) Int. Cl.³: **A 61 F 1/22**

(30) Priority: **28.04.80 US 144034**
**08.12.80 US 213944**

(43) Date of publication of application:
**04.11.81 Bulletin 81/44**

(84) Designated Contracting States:
**BE CH DE FR IT LI LU NL SE**

(71) Applicant: **MITRAL MEDICAL INTERNATIONAL, INC.**
**Suite 375 South Steele Street**
**Denver Colorado 80209(US)**

(72) Inventor: **Mayer, Louis Carl**
**8076 West Iliff Lane**
**Denver Colorado(US)**

(72) Inventor: **Goodenough, Samuel Henry**
**3366-C Cheltham Way**
**Dana Point California(US)**

(74) Representative: **Taylor, Derek George et al,**
**Mathisen, Macara & Co. Lyon House Lyon Road**
**Harrow, Middx. HA1 2ET(GB)**

(54) **Artificial heart valve.**

(57) A prosthetic heart valve has an annular body (21) and one or more leaflets (16) movable within the body between valve-open and valve-closed positions, the or each leaflet having lateral projections which engage in guide recesses, shaped as segments of a circle, which guide pivoted and limited translatory movement of the leaflet between such open and closed positions.

Fig. 2

EP 0 039 217 A1

# ARTIFICIAL HEART VALVE

This invention relates to artificial heart valves which can be transplanted into the mitral, aortic, tricuspid or pulmonic portions of the heart.

Prosthetic heart valves have been devised to the end of replacing defective natural heart valves and to simulate as closely as possible the operation of natural heart valves. Essentially, the natural valves in the human heart operate as a check valve capable of operating between open and closed positions in response to the flow of blood to and from the heart. It is essential that the artificial heart valve achieve the rapid response time of the natural heart valve to the reverse flow of blood in moving between the open and closed positions with a maximum opening to an angle in excess of 60° to the transverse axis of the valve for the passage of blood with a minimum of turbulence. The valve should have a low profile so as to prevent any possible interference with the ventricular septum as well as to minimize aortic obstruction and low cardiac output, and there should be a low pressure gradient between the upstream and downstream ends of the valve when the valve is in its open position. For instance, a pressure gradient on the order of 20 millimeters to 50 millimeters of mercury in a natural heart valve is symptomatic of defective functioning of the valve; whereas, the pressure gradient or drop of a properly functioning valve should be less than 10 millimeters of mercury.

It is therefore an object of the present invention to provide a novel and improved artificial heart valve in which one or more valving elements are movable between an open and closed position in response to the direction of fluid flow, are movable through a minimum angle between a fully closed and a fully opened position; and in the open position the leaflet or leaflets will permit substantially unrestricted flow of blood therethrough so as to minimize turbulence and promote laminar flow of the blood.

According to the present invention there is provided a prosthetic heart valve comprising an outer peripheral, generally annular body having an inner wall surface, at least one leaflet disposed in said body, said leaflet including opposed lateral edge surfaces and leading and trailing edge surfaces, and leaflet-supporting means operative to support said leaflet for movement in response to reversals in the direction of fluid flow through said body between an open position in which said leaflet is substantially parallel to fluid flow through said body and a closed position in which said leaflet extends angularly across said body, said leaflet-supporting means comprising lateral projections on opposed lateral edge surfaces of the leaflet and guide pocket means for each of said lateral projections, each said guide pocket means disposed in the inner wall surface of said body between said leading edge surface and said trailing edge surface of said leaflet, each said guide pocket means being arranged to receive one of said lateral projections whereby to guide pivotal and limited translatory movement of each said leaflet between said open and said closed positions in response to reversals in the direction of fluid flow through said body.

Forms of the invention are presented herein either for a mono-leaflet, bi-leaflet or tri-leaflet valve, respectively, in which the leaflets move in a radially outward direction away from the centre line of the valve into the open position, as opposed to moving toward the centre of the valve in the open position. In the mono-leaflet valve, the leaflet is supported in the closed position on a ledge of the valve body. The upstream side of the occluder leaflet is of a gently curved convex shape and the downstream side is a concave curve. This provides a significant increase in the flow area between the open occluder leaflet and the valve body on the side proximal to the projections and guide channels, otherwise referred to as the "minor orifice", when the leaflet is in the open position.

In the single leaflet construction, an optimum curvature is established for the guide members on the leaflet so that in their placement within and movement through the guide channels an optimum contact angle is established which will assure movement between precisely defined angles in the open and closed position notwithstanding the ability of the guide members to undergo translatory movement in combination with their pivotal movement through the guide channels.

These and other features of the present invention will become more readily understood from the following description together with the accompanying drawings in which:-

Figure 1 is a plan view of a bi-leaflet artificial heart valve as viewed from the downstream side of the valve;

Figure 2 is a cross-sectional view taken about lines 2-2 of Figure 1;

Figure 3 is a cross-sectional view taken about lines 3-3 of Figure 1;

Figure 4 is a view taken about lines 4-4 of Figure 2;

Figure 5 is a side view of the leaflet illustrated in Figures 1 and 2;

Figure 6 is a plan view of the leaflet shown in Figure 5;

Figure 7 is a cross-sectional view taken about lines 7-7 of Figure 5;

Figure 8 is a plan view of a tri-leaflet valve with one portion broken away to illustrate the channel for guiding movement of the leaflets therein;

Figure 9 is a front view of the valve construction shown in Figure 8;

Figure 10 is a cross-sectional view taken about lines 10-10 of Figure 8;

Figure 11 is a plan view of the valve body as shown in Figure 9 with a portion broken away to illustrate the configuration of one of the guide channels;

Figure 12 is a plan view of one of the occluder leaflets of Figure 9;

Figure 13 is a front view of the occluder leaflet shown in Figure 12;

Figure 14 is a cross-sectional view taken about lines 14-14 of Figure 13;

Figure 15 is a plan view of a preferred form of single leaflet construction;

Figure 16 is a cross-sectional view of the valve shown in Figure 15;

Figure 17 is a cross-sectional view taken about lines 17-17 of Figure 16;

Figure 18 is a cross-sectional view of a guide pocket taken about lines 18-18 of Figure 16;

Figure 19 is a plan view of the single leaflet illustrated in Figures 15 and 16;

Figure 20 is a side view of the leaflet shown in Figure 19; and

Figure 21 is a cross-sectional view taken about lines 21-21 of Figure 20.

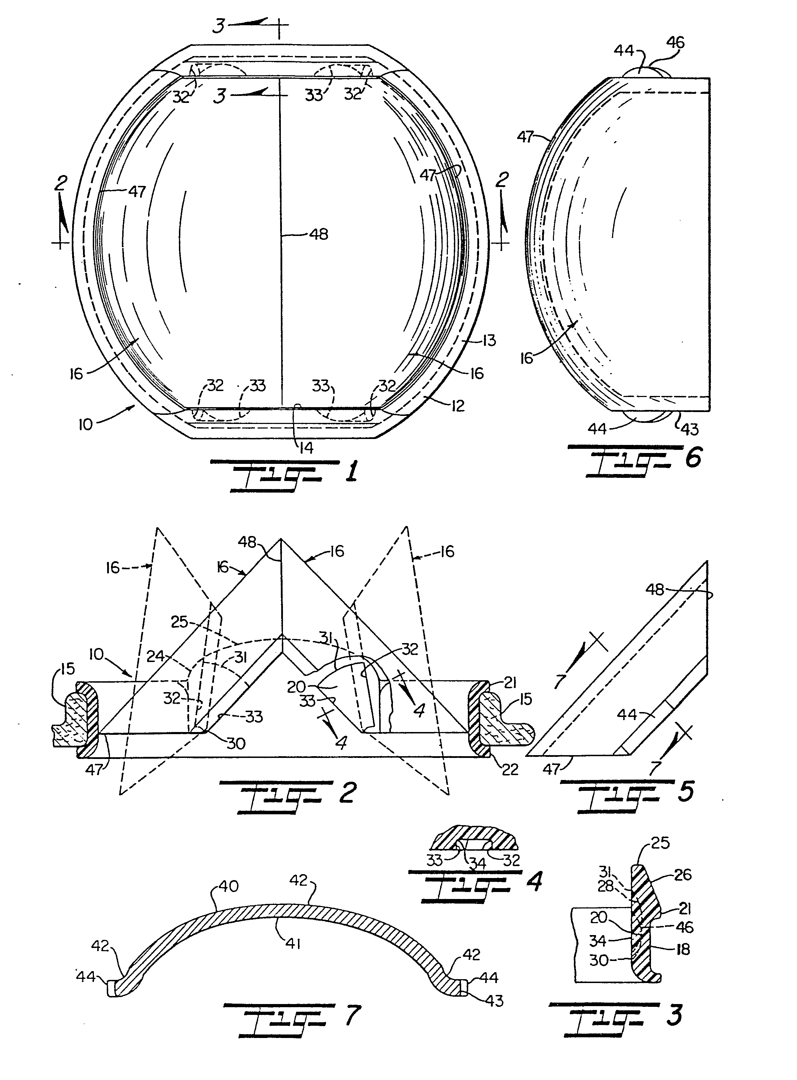

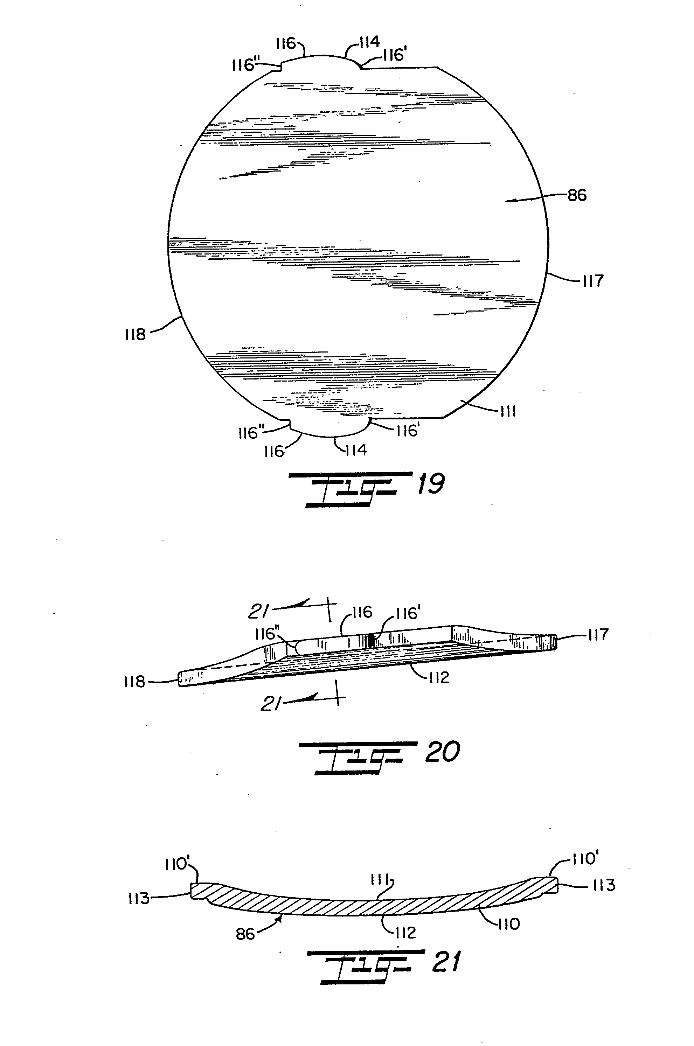

As illustrated in Figures 1 to 7, a bi-leaflet valve 10 is comprised of a generally annular valve body 12 having diametrically opposed arcuate wall sections 13 interconnected by straight wall sections 14, and the valve body is surrounded by an outer concentric suture ring 15. Valve members or occluder leaflets 16 are disposed for pivotal movement within the valve body in a manner hereinafter described.

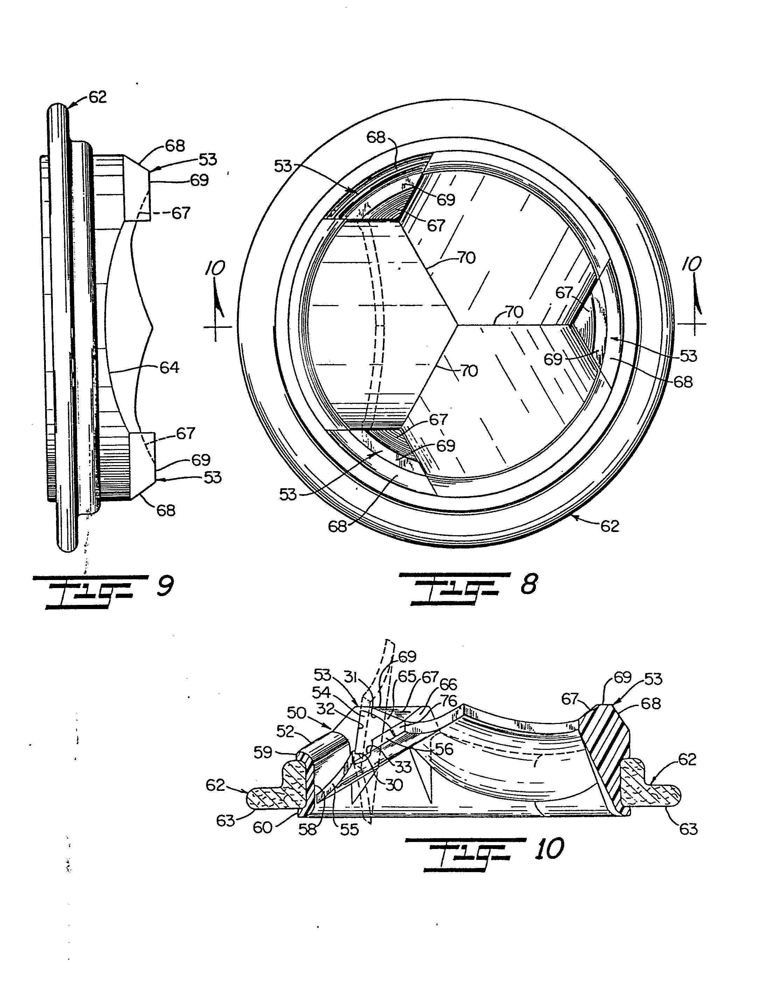

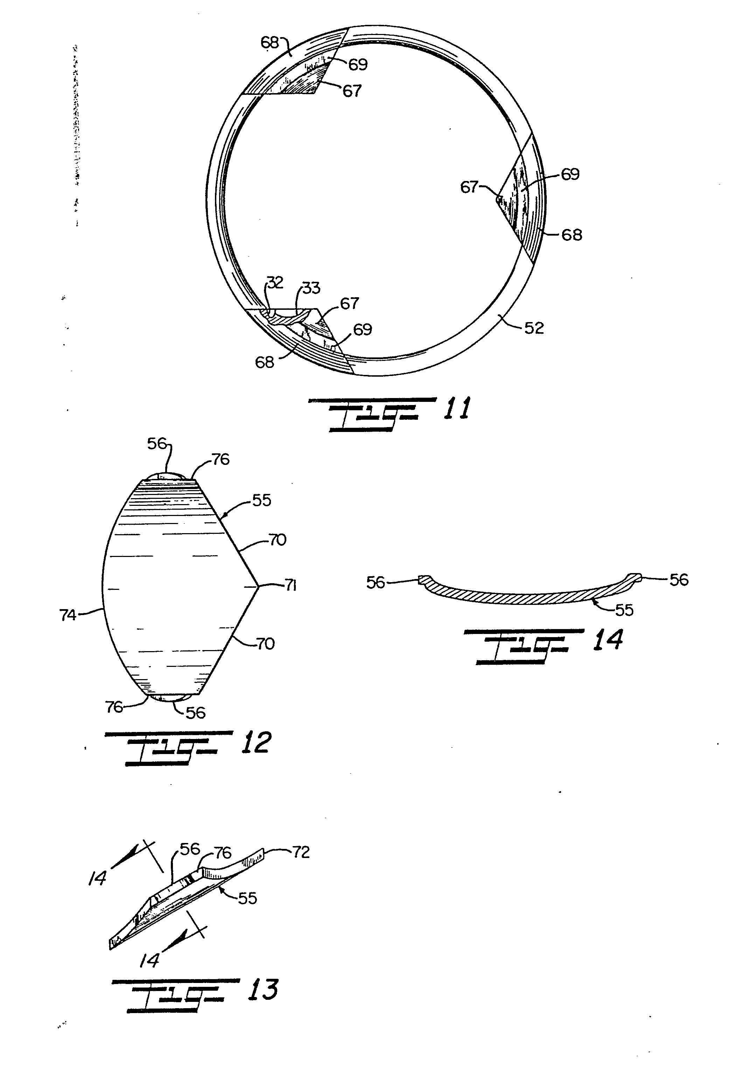

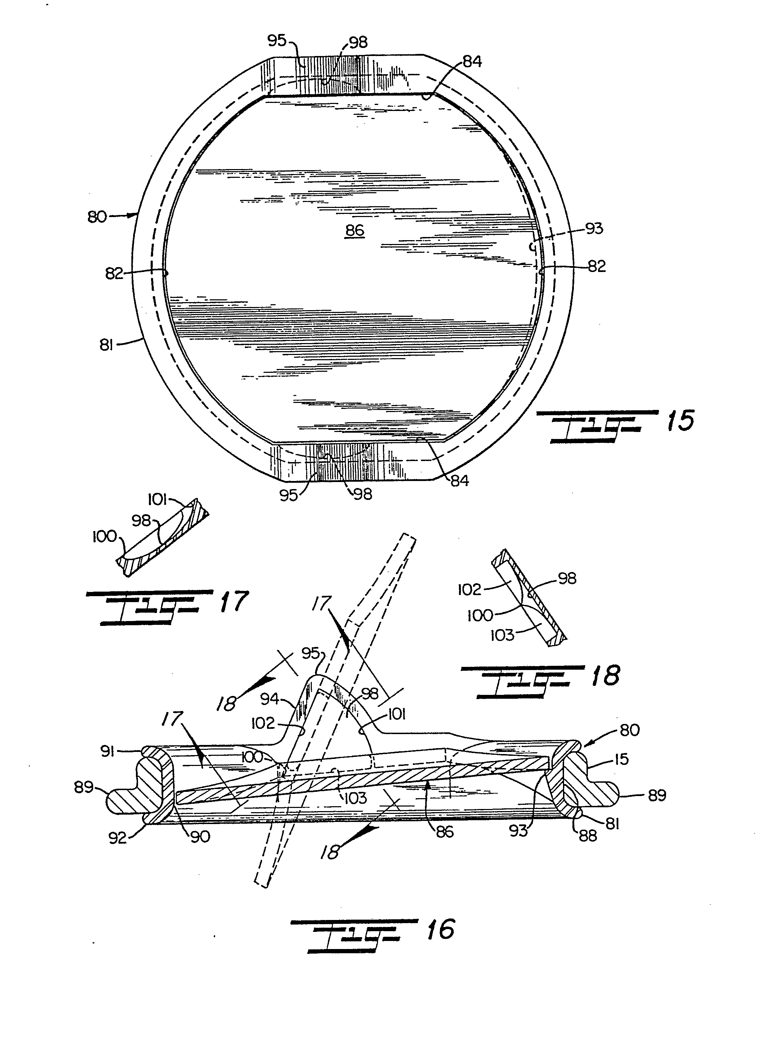

Both the arcuate wall sections 13 and the straight wall sections 14 are of generally channel-shaped cross-sectional configuration, as shown in Figures 3 and 4, so as to define a generally concave, channel-shaped external wall surface 18 which is adapted to receive the suture ring 15, the suture ring provided with an outwardly projecting collar 18 to facilitate implantation of the valve into the heart, for instance, as a substitute for the mitral valve. A modified type of suture ring, not shown, is employed when the valve is implanted in place of the aortic valve.

An inner wall surface 20 of the valve body 12 is flat throughout its greater height except for the downstream and upstream ends 21 and 22 which flare outwardly to form the external wall 18. In addition, each straight wall section 14 has an axial extension 24, each extention forming a direct continuation of the inner wall surface 20 in a downstream direction and terminating in a convex downstream edge 25. Each extension 24 is symmetrically formed about the centre of its associated straight wall section and has an external inclined wall surface 26 which converges upwardly from the upper end 21 into the convex edge 25 of the extension.

A pair of guide pockets in the form of shallow recesses or channels 28 are formed at opposite ends of each straight wall section 14 and, as illustrated in Figures 2 to 4, each channel is of a height to project from a point adjacent to upstream or lower edge 22 of the wall section to the downstream or convex edge 25 of the extension wall 24. As viewed in Figure 2, each guide pocket or channel 24 is of generally triangular configuration which is truncated by the intersection of a relatively short upstream edge 30 with divergent side edges 32 and 33. The downstream edge 30 is of a width just greater than the width of the guide
member to be described for the associated leaflet, and a
relatively wide downstream edge 31 is of curved or arcuate
configuration. In the preferred form, the divergent edge
32 is disposed at an angle on the order of 5° to 9° to the
longitudinal axis of the valve while the edge 33 which
establishes the closed position of the valve, is disposed
at an angle on the order of 45° to the longitudinal axis.
The inner wall or bearing surface 34 of each channel is
curvilinear having an elliptical intermediate wall surface
which verges into sloping or gently curving surfaces at
the outer surrounding edges 30 and 31, as shown in
Figure 3, but is flat across its width and terminates in
the more sharply angled edges 32 and 33, as shown in
Figure 4.

The occluder leaflets 16 are mounted for pivotal movement
within the channel 28 between a closed position as illustrated in full in Figures 1 and 2 and an open position as
shown dotted in Figure 2. The leaflets 16 are of corresponding size and configuration and, as shown in more
detail in Figures 5 to 7, each leaflet has a generally
shovel-shaped major wall portion 40 which is of generally
elliptical configuration with a concave surface 41 on one
side and convex surface 42 on the opposite or downstream
side. The elliptical wall portion terminates on opposite
sides in reversed curved sections which flatten or
straighten into lateral edges or skirts 42, the skirts
projecting a limited distrnace in opposite directions to
one another. Each skirt 42 terminates in a straight edge
43, and a slide control member 44 protrudes away from the
straight edge, the slide control member 44 being somewhat
elongated as illustrated in Figure 5 and having an external
curved or convex surface 46 corresponding to the curvature
of the bearing surface 34 in its associated channel 28.

As viewed from Figure 5, each leaflet can be further
characterized as being in the essential form of a
triangle having a hypoteneuse extending centrally of
the major wall portion of the leaflet and equilateral
side edges 47 and 48 which define the leading or upstream
edge of the leaflet and the trailing or downstream edge
of the leaflet, respectively. The triangle as described
is truncated by the intersection of the sides 47 and 48
with the skirts 42, the skirts 42 extending between the
sides 47 and 48 parallel to the hypoteneuse or mid-section
of the wall portion. Each upstream edge 47, as viewed in
Figure 6, is formed on a radius of curvature corresponding
to that of a curved wall section 13 of the annular body
and intersects the straight edged skirts 42 at a point.
corresponding to the straight wall sections 14 of the body.
In turn, the downstream edge 48 of each leaflet is movable
into flush engagement with a corresponding edge 48 of the
opposite leaflet when the leaflets move into the closed
position, as illustrated in full in Figure 2, with the
upstream edges 47 movable into contact with the arcuate
wall sections 13 of the body.

Considering the relationship between the slide control
members 44 and their respective channels 28, it will be
noted from Figures 1 and 2 that when the leaflets are
disposed in their closed position, each slide control
member 44 will extend substantially at a 45° angle to the
longitudinal axis of the body and parallel to the inner
side edge 33 of its associated channel. Each slide control
member is of a length corresponding to the length of the
longer edges 32 and 33 but is of a width less than the
width between the side edges 32 and 33 at their narrowest
point along the upstream edge 30. In this way, the slide control member does not have a fixed point of rotation as
it advances through the channel but is free to undergo a
combination of sliding and pivotal movement as it advances
from the closed position to the open position adjacent to
or abutting the opposite side edge 32 so as to achieve
most effective washing action within the guide channels or
pockets 28 as the leaflets are controlled by fluid pressure
in their movement between the open and closed positions.
Moreover, the slide control members are curved only in a
lengthwise direction and are flat across their width so as
to correspond  to the flat bearing surface of its associated channel and are of a depth to be fully seated within
each of the respective channels 28 as illustrated in
Figure 3.

When liquid or fluid under pressure is applied to the upstream or concave surfaces 41 of the leaflets, the opening
pressure against the leaflets will cause the slide control
members 44 to be advanced from the position shown in full
in Figure 2 to the dotted line position as described.
Again, the slide control members will undergo both a sliding or translational movement together with a swinging or
pivotal movement through the channels with the upstream end
of the slide control member free to slide along the upstream
edge 30 and the downstream end of the member being free to
advance with respect to the downstream edge 31 of the
channel until the member advances from a position abutting
the inner side edge 33 to an open position abutting the
outer side edge 32.  In the open position, the leaflets will
have been advanced from a substantially 45° angle to an
approximate 5° angle to the longitudinal axis, or just less
than parallel to the longitudinal axis.  In this relation,
the leaflets will have moved in an outward direction toward
the inner wall surfaces of the body so as to form an open passageway for the liquid or fluid to flow therethrough and which passageway is free of any obstructions other than the limited projection of the skirts 42 along the straight wall sections. By eliminating any fixed axis or point or rotation between the slide control members and channels, the blood or other liquid flowing therethrough can effectuate more complete washing action of the channels which could otherwise tend to collect the blood or other liquid. Moreover, the blood will be free to pass to a limited extent between the skirt portions 32 and straight wall sections 14 so as to have the effect of a washing action on the mating surfaces between the slide control members 44 and associated channels 28. When the direction of liquid or fluid flow is reversed, the pressure will be shifted to bear against the convex or downstream surface 42 of each leaflet so as to cause the slide control members 44 to undergo reverse movement through the channels in an upstream or radially inward direction until the edges 48 of the leaflets return into closed relation to one another so as to effectively check the flow in the reverse direction. Formation of the slide control members with respect to the pockets 28 in the manner described will eliminate any tendency of the leaflets to move beyond the open position shown in Figure 2 but at the same time obviates the use of separate limit stops or projections to regulate the degree of opening or closing movement. Still further, the fan-shaped configuration of the channels 28 in the extension walls achieves a low profile valve with rapid response time in opening and closing of the valve in response to reversals in fluid or liquid flow therethrough.

In the modified form of invention, a tri-cuspid or tri-leaflet valve is designated at 50 having an annular body 52 provided with extension walls 53 at equally spaced circumferential intervals in which are positioned guide
pockets or channels 54 to guide and support occluder
leaflets or valve members 55.  In the tri-leaflet form,
a series of three leaflets are positioned within the body,
each being of corresponding size and width having slide
control members 56 on opposite sides of each leaflet which
are movable through the guide channels 54 between a closed
position as in Figure 8 and an open position as in Figure 10.

The annular body 52 is once again of generally channel-shaped
configuration having a straight inner wall surface 58 bounded
by flared ends 59 and 60 on its downstream and upstream sides,
respectively.  A suture ring 62 is positioned within the
channel formed by the flared ends 59 and 60 and is provided
with a collar 63 projecting in a radial outward direction
from the body to facilitate implantation as a mitral valve in
the manner described in the preferred form.  Although the upstream end 60 of the body is flat, the downstream end 59 is
somewhat scalloped or concave, as indicated at 64 in Figure 9,
in its extension between the extension walls 53.  The extension walls 53 in turn project in an axial direction away from
the main wall of the body and are of generally triangular
cross-section as viewed in Figure 8 to present radially inwardly inclining, equilateral side surfaces 65 and 66 which
intrude for a limited distance from the inner straight wall
surface of the body.  As a result, the extension walls are
formed so as to be of gradually increased thickness in
extending in a downstream direction away from the body 52
and terminate in inclined downstream surfaces 67 and 68
which are joined or interconnected by a horizontal edge 69.

Each of the channels 54 is formed in the sides 65 and 66 of
each extension wall 53 and correspond in construction as
well as intended function with the channels 28 of Figures 1
to 7.  Accordingly, like parts or surfaces of the channels 54
are correspondingly enumerated to that of the preferred form
of Figures 1 to 7.

As shown in Figures 12 to 14, each occluder leaflet or
valve member 55 is of corresponding size and configuration
and is dimensioned for insertion between confronting sides
65 and 66 of adjacent extension walls so as to move between
a closed position as shown in full in Figure 8 and an open
position as shown in dotted form in Figure 10.  Each
occluder leaflet can be generally characterized as being
shovel-shaped and of a somewhat flatter curved configuration than the preferred form of bi-leaflet arrangement shown
in Figures 1 to 7.  Each leaflet has leading straight edges
70 which converge to an apex or point 71 and are somewhat
beveled or tapered as indicated at 72 so as to move into
flush engagement with adjoining leading edges of the other
leaflets in the closed position shown in Figure 8.  In
addition, each leaflet has a beveled trailing edge 74 which
is formed on a radius of curvature corresponding to the
inner wall surface 58 of the body with opposite ends of the
trailing edge 74 intersecting straight lateral edges 76 on
opposite sides of each leaflet.  A slide control member 56
protrudes from the lateral edge 76 on each side of the
leaflet and is of generally lobe-shaped configuration
corresponding to the configuration of the slide control
members 44 of the preferred form.  The lateral edges 76 are
of a length so as to be free to slide or advance along the
correspondingly flat surfaces of the sides 65 or 66 of the
extension walls with the slide control member 56 movable
through a guide channel 54 in controlling opening and closing movement of the leaflets.

It will be noted in the tri-leaflet configuration, that the
leading edges of the leaflets 55 define a generally Y-
shaped parting line in the closed position; and similarly,
each pair of adjoining leaflets define a Y-shaped parting
line with a common extension wall, as seen from a consideration of Figure 8.

A single leaflet valve 80 as shown in Figures 15 to 21
includes an annular valve body 81 having diametrically
opposed, arcuate wall sections 82 interconnected by
straight wall sections 84, and a suture ring 15 corresponding to that illustrated in Figure 1, is mounted in
outer surrounding relation to the valve body. A single
occluder leaflet 86 is disposed for pivotal movement within the valve body between a closed position, as illustrated
in full in Figure 16, extending at an acute angle to a
plane through the valve body and an open position extending
at an acute angle to the longitudinal axis of the valve
body, shown dotted in Figure 16.

Referring in more detail to the construction of the valve
body 81, both the arcuate wall sections 82 and straight
wall sections 84 again define a general channel-shaped
member having an external concave or channel-shaped external
wall surface 88 adapted to receive the suture ring 15, and
the ring includes an outwardly projecting collar 89 for
implantation into the heart, for instance, as a substitute
for the mitral valve. The suture ring may be modified
according to the particular locus of implantation. An inner
wall surface 90 of the body 81 is relatively flat throughout
its greater height, except for the downstream and upstream
ends 91 and 92 which flare outwardly to form the external
wall 88, and an arcuate ledge 93 is formed in the inner
wall to extend circumferentially around one of the arcuate
wall sections 82, the ledge facing in the downstream direction and adapted to serve as a supporting surface for the
leaflet 86 in a manner to be described. As shown in
Figure 16, the ledge 93 slopes upwardly from its midpoint,
or in other words is given a slight concavity so as to be
complementary to the convex upstream surface of the leaflet
as hereinafter described. Moreover, each straight wall
section 84 has an axial extension 94 which forms a direct axial continuation of the valve body in a downstream
direction and terminates in a generally convex or triangular edge 95. Each extension 94 is correspondingly
disposed in offset relation to the centre line of the
valve body so as to extend upwardly from a point relatively
near one end of each straight wall section and with the
extensions being aligned in opposed confronting relation to
one another.

Guide pockets are defined by shallow recesses or bearing
surfaces 98, each pocket being of a height to project from
a point adjacent to a downstream edge of a wall section 84
to the downstream edge or apex of the axial extension.
Each guide pocket 98 is of generally triangular configuration having an upstream end 100 and a curved downstream
edge 101 interconnected by divergent side edges 102 and 103.
The upstream end 100 is at the intersection of the side
edges 102 and 103 or may by truncated somewhat as described
with reference to the forms illustrated in Figures 1 to 14.
In this form, the outer divergent side edge 102 is disposed
at an angle on the order of 25° to the longitudinal axis of
the valve while the opposite or inner edge 103 is disposed
at an angle on the order of 80°-85° to the longitudinal axis.
The inner bearing surface 98 of each guide pocket is of
generally curvilinear configuration having a generally concave intermediate wall surface which is in the form of a
gently curving ellipse in a direction between the upstream
and downstream edges 100 and 101, but is flat in its extension across the width between the divergent edges 102 and
103.

The single leaflet 86 is mounted for pivotal movement within the valve body between a closed position as illustrated
in full in Figure 16 and an open position, as shown dotted
in Figure 16. As shown in Figure 19 to 21, the leaflet has a generally shovel-shaped major wall portion 110 which is
of concavo-convex configuration and provided with a concave surface 111 on its downstream side and a convex
surface 112 on its opposite or upstream side. The leaflet
terminates on opposite sides in flat side extensions projecting a limited distance in opposite directions to one
another so as to terminate in straight edges 113. A guide
or slide control member 114 protrudes from each of the
straight edges 113 and is somewhat elongated as illustrated
in Figure 15 with a gently curved or convex edge 116 terminating in a squared or straight edge 116' and a rounded
edge 116'' at its juncture with the straight edge 113. The
guide member 114 is dimensioned such that when inserted
into a respective guide pocket, the straight edges 116' and
116'' will bear slightly against the opposed upstream and
downstream edges 100 and 101 with the generally convex edge
116 seated on the bearing surface 104 in the guide pocket.
Most desirably, the convex edge is given an elliptical curvature along its length from the leading edge portion 116'
rearwardly which corresponds to the elliptical curvature of
the bearing surface 104; however, the trailing edge 116''
of the convex edge 113 is transversely rounded symmetrically about the centre line of the slide member 114 to
permit it to pivot smoothly between the open and closed
positions and will enable the slide members 14 to move
freely into flush engagement with the divergent sides 103
and 104 without interference between the elliptical bearing surface 104 and the convex edge 116. As a result, the
guide pockets 98 will establish a precisely controlled
angle of the leaflets in the open position and avoid any
shifting or misalignment between the trailing edge 116''
and upstream edge 100 at the narrow end of the guide
pocket.

In the construction of the single leaflet, the lateral edges 113 are sized to correspond to or be slightly less than the length of the straight wall sections 84, and arcuate leading and trailing edge surfaces 117 and 118 correspond to the arcuate wall sections 82 of the valve body, except for allowance of a slight gap between the leading edge 118 and the arcuate wall section 82, as shown in Figures 15 and 16. Here, the single leaflet construction has a gentler slope or curvature than that of the bi-leaflet or tri-leaflet constructions and for instance is formed on a curvature between the lateral edges on the order of a 6.5° ellipse. Thus, when the guide members 114 are inserted into their respective guide pockets, the leaflet is controlled in its movement between a closed position in which the leading edge 117 engages the arcuate ledge 93 and an open position, again as shown dotted in Figure 16, in which the leaflet is disposed more nearly parallel to the longitudinal axis of the valve body. By virtue of the slight angle or inclination of the leaflet 86 in the closed position, the leaflet 93 slopes at a corresponding angle radially inwardly and in an upstream direction so that the leading edge 117 will advance into substantially flush relation to the ledge 93. However, owing to the gap formed between the leaflet and arcuate wall section 82 adjacent to the ledge 93 a controlled leakage of blood past the leaflet is permitted in the closed position just sufficient to keep the blood agitated and avoid any tendency to clot or coagulate.

When blood flows under pressure into the heart and is applied to the upstream or convex surface 112 of the leaflet, the opening pressure against the leaflet will cause its slide control members 114 to undergo a combination of sliding and pivotal movement from the position shown in full in Figure 16 to the dotted line position. In undergoing both a limited sliding or translational movement along with a pivotal movement through the guide pockets, the slide control members 114 will advance from a position abutting the divergent edge 103 to an open position abutting the edge 102. In moving to the open position, the leaflet will have undergone a movement of substantially 60° toward the arcuate wall section of the valve body so as to form open passageways for the blood to flow both along the major and minor orifices and which passageways are free of any obstructions along opposed surfaces of the leaflets. Once again, by eliminating a fixed axis or point of rotation between the guide members and guide pockets, the blood or other liquid flowing therethrough can effectuate more complete washing action of the guide pockets while being free to pass to some extent between the side extensions and straight wall sections, thereby having the effect of a washing action on the mating surfaces between the slide control members 114 and guide pockets 98. When the direction of flow of blood is reversed, the fluid pressure will be applied to the concave surface 111 of each leaflet so as to cause the guide members 114 to undergo reverse movement through the guide pockets in an upstream direction until the leading edge of the leaflet returns into closed relation to the supporting ledge 93 and effectively checks the flow of blood in a reverse direction.

It is therefore to be understood that while preferred and modified forms of the the invention have been set forth and disclosed herein that various modifications and changes may be made without departing from the spirit and scope of the invention as defined by the appended claims.

## CLAIMS

1.    A prosthetic heart valve characterised by an outer
peripheral, generally annular body having an inner wall
surface, at least one leaflet disposed in said body, said
leaflet including opposed lateral edge surfaces and leading
and trailing edge surfaces, and leaflet-supporting means
operative to support said leaflet for movement in response
to reversals in the direction of fluid flow through said
body between an open position in which said leaflet is substantially parallel to fluid flow through said body and a
closed position in which said leaflet extends angularly
across said body, said leaflet-supporting means comprising
lateral projections on opposed lateral edge surfaces of the
leaflet and guide pocket means for each of said lateral
projections, each said guide pocket means disposed in the
inner wall surface of said body between said leading edge
surface and said trailing edge surface of said leaflet,
each said guide pocket means being arranged to receive one
of said lateral projections whereby to guide pivotal and
limited translatory movement of each said leaflet between
said open and said closed positions in response to reversals in the direction of fluid flow through said body.

2.    A prosthetic heart valve according to claim 1,
characterised by said opposed lateral edge surfaces being
flat and extending substantially parallel to one another
and movable into substantially sealed relation with
respect to a correspondingly flat inner wall surface portion of said body when said leaflets are advanced to the
closed position, and said leaflet-supporting means being
elongate in a direction parallel to said lateral edge
surfaces.

3. A prosthetic heart valve according to claim 1 or claim 2 characterised by said guide pocket means each being in the form of a shallow guide lane channel formed as a shallow depression in a flat inner wall surface portion of said body, said guide lane channel of each pair of guide lane channels on diametrically opposite sides of said body diverging away from one another at an acute angle from a point relatively near the leading edge surface of the, or each respective, leaflet.

4. A prosthetic heart valve according to claim 3 characterised by said opposed lateral edge surfaces of the or each leaflet being in the form of a skirt, said skirts extending substantially parallel to one another and to said flat inner wall surface portions of said body in a direction upstream of the direction of flow through said valve.

5. A prosthetic heart valve according to any preceding claim, characterised by said guide pocket means each being in the form of a shallow multi-sided guide channel of triangular configuration with opposite side edges divergent in a downstream direction and said lateral projections being of a length to be received in an associated channel and slidable through said channel between said opposite side edges.

6. A prosthetic heart valve according to any preceding claim, characterised by there being a pair of leaflets, each pair of said guide channels on opposite sides of each said leaflet being formed in an axially directed extension of said valve body symmetrically about a plane passing through the longitudinal axis of said valve body and intermediate each channel of a pair.

7.    A prosthetic heart valve according to any one of claims 1 to 5, characterised by said valve body having an inner wall surface provided with a supporting ledge and the leading edge of said leaflet being movable into contact with said supporting ledge in the closed position.

8.    A prosthetic heart valve according to any preceding claim, characterised by said leaflet having a major wall portion of concavo-convex configuration with its convex surface on the upstream side and having curved leading and trailing edge surfaces substantially corresponding to the curvature of the inner wall surface of said annular valve body.

9.    A prosthetic heart valve according to claim 7, characterised by said supporting ledge extending circumferentially of said inner wall surfaces for a distance corresponding to said leading edge surface and having a concave surface complementary to the convex surface of said leaflet.

Fig 1

Fig 6

Fig 2

Fig 5

Fig 4

Fig 7

Fig 3

Fig 9

Fig 8

Fig 10

_Fig_ 11

_Fig_ 12

_Fig_ 14

_Fig_ 13

_Fig_ 15

_Fig_ 17

_Fig_ 18

_Fig_ 16

Fig 19

Fig 20

Fig 21

## EUROPEAN SEARCH REPORT

European Patent Office

Application number

EP 81 30 1815.7

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| X | GB - A - 2 018 396 (CARBOMEDICS INC.) <br> * whole document * <br> -- | 1,2,3, 6 | A 61 F 1/22 |
| X | GB - A - 2 007 333 (ST JUDE MEDICAL INC.) <br> * whole document * <br> -- | 1,2,3, 6 | |
| | US - A - 4 114 202 (H.A. ROY et al.) <br> * fig. * <br> -- | 1,2 | |
| A | FR - A1 - 2 331 997 (RHONE-POULENC INDUSTRIES) <br> * fig. 6 to 9 * <br> & GB - A - 1 565 810 <br> -- | 1 | TECHNICAL FIELDS SEARCHED (Int. Cl.³) <br><br> A 61 F 1/00 |
| A | GB - A - 1 167 329 (J. WADA) <br> * fig. 11 to 13 * <br> -- | 1 | |
| A | US - A - 4 078 268 (Z.C. POSSIS) <br> * fig. 3 to 5 * <br> -- | 1 | |
| A | US - A - 3 546 711 (J.C. BOYROS) <br> * fig. 2 to 4 * <br> -- | 1,7,9 | CATEGORY OF CITED DOCUMENTS <br><br> X: particularly relevant <br> A: technological background <br> O: non-written disclosure |
| P | EP - A1 - 0 023 797 (HEMEX INC.) <br> * whole document * <br> ---- | 1 | P: intermediate document <br> T: theory or principle underlying the invention <br> E: conflicting application <br> D: document cited in the application <br> L: citation for other reasons |

&: member of the same patent family, corresponding document

The present search report has been drawn up for all claims

| Place of search | Date of completion of the search | Examiner |
|---|---|---|
| Berlin | 22-07-1981 | KANAL |

EPO Form 1503.1 06.78